(19) Europäisches Patentamt
European Patent Office
Office européen des brevets

(11) EP 3 778 540 A1

(12) EUROPEAN PATENT APPLICATION
published in accordance with Art. 153(4) EPC

(43) Date of publication:
17.02.2021 Bulletin 2021/07

(21) Application number: 19777523.2

(22) Date of filing: 20.03.2019

(51) Int Cl.:
*C07C 39/16* (2006.01) *C07C 37/20* (2006.01)
*C07C 39/17* (2006.01) *C07D 303/30* (2006.01)
*C08G 8/20* (2006.01) *C08G 59/32* (2006.01)

(86) International application number:
PCT/JP2019/011657

(87) International publication number:
WO 2019/188645 (03.10.2019 Gazette 2019/40)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 27.03.2018 JP 2018059219
25.09.2018 JP 2018178808

(71) Applicant: SEKISUI CHEMICAL CO., LTD.
Osaka-shi
Osaka
530-8565 (JP)

(72) Inventor: CORNILLE, Adrien
Tsukuba-shi, Ibaraki 300-4292 (JP)

(74) Representative: Ter Meer Steinmeister & Partner
Patentanwälte mbB
Nymphenburger Straße 4
80335 München (DE)

# (54) BIS-PROPYLCATECHOL, METHOD FOR PRODUCING BIS-PROPYLCATECHOL, RESIN COMPOSITION CONTAINING BIS-PROPYLCATECHOL, RESIN CURED PRODUCT OBTAINED BY CURING RESIN COMPOSITION CONTAINING BIS-PROPYLCATECHOL, EPOXIDIZED BIS-PROPYLCATECHOL, METHOD FOR PRODUCING EPOXIDIZED BIS-PROPYLCATECHOL, CURABLE RESIN COMPOSITION CONTAINING EPOXIDIZED BIS-PROPYLCATECHOL, AND RESIN CURED PRODUCT OBTAINED BY CURING CURABLE RESIN COMPOSITION CONTAINING EPOXIDIZED BIS-PROPYLCATECHOL

(57) A bis-propylcatechol having a structure represented by formula (1):

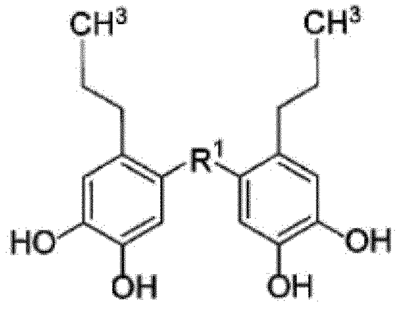

・・・ (1)

wherein $R^1$ is a divalent aliphatic hydrocarbon group having two or more carbon atoms or a divalent aromatic hydrocarbon group.

EP 3 778 540 A1

## Description

TECHNICAL FIELD

The present invention relates to a bis-propylcatechol obtainable from lignocellulosic biomass and a method for producing a bio-based bis-propylcatechol and, moreover, a resin composition and a cured resin product each containing the bio-based bis-propylcatechol.

In addition, the present invention relates to an epoxidized bis-propylcatechol obtained from the bis-propylcatechol and a method for producing an epoxidized bis-propylcatechol and, moreover, a curable resin composition and a cured resin product each containing the epoxidized bis-propylcatechol.

The present application claims priority on Japanese Patent Application No. 2018-059219, filed March 27, 2018, and Japanese Patent Application No. 2018-178808, filed September 25, 2018, the contents of which are incorporated herein by reference.

Background Art

Currently, 2,2-Bis(4-hydroxyphenyl)propane generally referred to as "Bisphenol A (hereinafter sometimes referred as BPA)" is a most representative bis-phenolic monomer available in the market, which is used for producing polymers such as polycarbonates and epoxy resins. Specifically, BPAmakes up more than 77% of the bis-phenolic monomers demanded globally. However, the supply of BPA is heavily dependent on petro-based benzene markets.

In addition, recently, United-States Food and Drug Administration has prohibited the use of BPA-based materials in packaging for child products because of its endocrine disruptor activity inducing a reduced fertility and potentially cancers. In 2015, France also prohibited BPA for all food-contact packaging.

Also in Japan, the Food Sanitation Act restricts the elution amount of BPA from polycarbonate instruments, containers and packaging as well as the BPA contents of materials. The envisaged substitutes for BPA are 4,4'-methylenediphenol (Bisphenol F) and 4,4'-sulfonyldiphenol (Bisphenol S) and the like. However these chemicals are also dependent on petro-based benzene and, as concluded from a recent literature review, endocrine disruptors (see Non Patent Document 1).

For these reasons, bio-based substitutes for BPA have been drawing attention. One candidate compound as such substitutes is bis-propylcatechol. This compound can be produced from 4-propylcatechol which itself is produced by a known method of demethylation of bio-based 2-methoxy-4-propylphenol (see Non Patent Document 2).

This bis-propylcatechol is expected to be applicable to the production of bio-based polymers. The above-mentioned 2-methoxy-4-propylphenol can be obtained from extraction and selective depolymerization (hydrogenation) of lignocellulosic biomass (see Patent Document 1). Non Patent Document 3 discloses a study on the synthesis of bis-propylcatechol for the production of epoxidized bis-propylcatechol. Non Patent Document 3 discloses that the formulation of bio-based epoxy resins was achieved.

To obtain bio-based bis-propylcatechol compound, Non Patent Document 3 used the successive reaction between 2-methoxy-4-propylphenol and formaldehyde to dimerize 2-methoxy-4-propylphenol, followed by demethylation of the thus obtained bis-(2-methoxy-4-propylphenol) in presence of aqueous solution of hydrobromic acid.

However, in this process, the harmful formaldehyde is used to access to this bis-propylcatechol. In addition, the epoxidized compound, which is obtained after the reaction between epichlorohydrin and bis-propylcatechol, possesses a methylene group between the two phenolic moieties. Once this epoxidized bis-propylcatechol is cured in material formulation, this methylene group decreases the thermal and mechanical properties to lower levels as compared to BPA-based material.

Specifically, it is considered that the methylene group linking the two phenolic moieties in bis-propylcatechol allows for higher freedom of rotation of the phenolic moieties than allowed by the isopropylidene group linking the two phenolic moieties in BPA, which works to the detriment of the thermal and mechanical properties of cured resin product.

Citation List

Patent Literature

[Patent Document1] WO2015061802A1

Non-patent Literature

[Non Patent Document 1] Rochester, J. R.; Bolden, A. L., Bisphenol S and F: A systematic review and comparison of the hormonal activity of Bisphenol A substitutes. Environ Health Perspect 2015, 123 (7), 643-50.

[Non Patent Document 2] Zhao, S.; Abu-Omar, M. M., Renewable Thermoplastics Based on Lignin-Derived Polyphenols. Macromolecules 2017, 50 (9), 3573-3581.

[Non Patent Document 3] Zhao, S.; Abu-Omar, M. M., Renewable epoxy networks derived from lignin-based monomers: effect of cross-linking density. ACS Sustainable Chem. Eng. 2016, 4 (11), 6082-6089.

DISCLOSURE OF INVENTION

Technical Problem

The present invention has been made in view of the aforementioned situation, and the object of the present invention is to provide a bis-propylcatechol which is derivable from renewable resources such as lignocellulosic biomass and without using harmful formaldehyde, and which can be converted into an epoxidized bis-propylcatechol with excellent thermal and mechanical properties comparable to conventional BPA-based epoxy resins.

Solution to problem

The bis-propylcatechol, the method for producing the bis-propylcatechol, the resin composition and cured resin product each containing the bis-propylcatechol, the epoxidized bis-propylcatechol, the method for producing the epoxidized bis-propylcatechol, and the curable resin composition and cured resin product each containing epoxidized bis-propylcatechol according to the present invention are as enumerated below in [1] to [23].

[1] A bis-propylcatechol having a structure represented by formula (1):

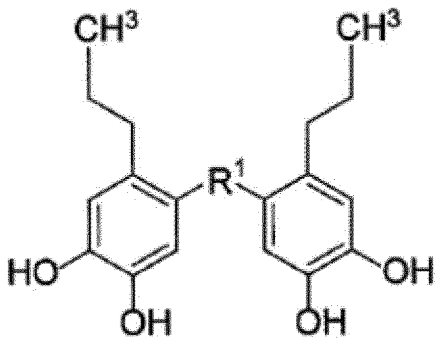

・・・(1)

wherein $R^1$ is a divalent aliphatic hydrocarbon group having two or more carbon atoms or a divalent aromatic hydrocarbon group.

[2] The bis-propylcatechol according to [1], wherein $R^1$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

[3] The bis-propylcatechol according to [1], wherein $R^1$ is a divalent hydrocarbon group represented by $-CHR^5-$ wherein $R^5$ represents a monovalent hydrocarbon group.

[4] The bis-propylcatechol according to [1], wherein $R^1$ is a divalent aromatic hydrocarbon group represented by formula (2):

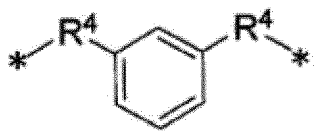

・・・(2)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure, and * represents a binding site to a benzene ring in the propylcatechol structure in the above formula (1).

[5] The bis-propylcatechol according to [4], wherein each of $R^2$ and $R^3$ represents a methyl group.

[6] The bis-propylcatechol according to [1] or [3], wherein $R^1$ is a divalent hydrocarbon group represented by formula (3):

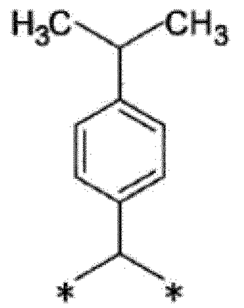
· · · (3)

wherein * represents a binding site to a benzene ring in the propylcatechol structure in the above formula (1).

[7] The bis-propylcatechol according to [1], wherein $R^1$ is a divalent aliphatic hydrocarbon group represented by formula (4):

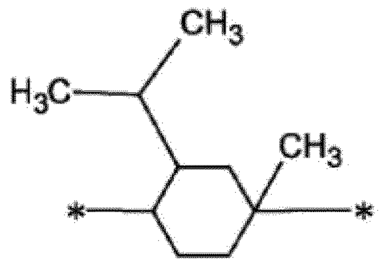
· · · (4)

wherein * represents a binding site to a benzene ring in the propylcatechol structure in the above formula (1).

[8] The bis-propylcatechol according to [1] or [3], wherein $R^1$ is a divalent aliphatic hydrocarbon group represented by formula (5):

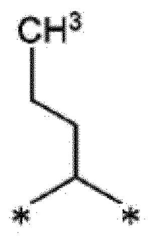
· · · (5)

wherein * represents a binding site to a benzene ring in the propylcatechol structure in the above formula (1).

[9] The bis-propylcatechol according to [1] or [3], wherein $R^1$ is a divalent aliphatic hydrocarbon group represented by formula (6):

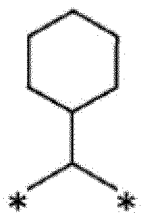

(6)

wherein * represents a binding site to a benzene ring in the propylcatechol structure in the above formula (1).

[10] A method for producing the bis-propylcatechol of any one of [1] to [9], comprising reacting a propylcatechol and at least one reactant selected from the group consisting of a ketone, an alcohol, an aldehyde and a mono-terpene.

[11] A resin composition comprising the bis-propylcatechol according to any one of [1] to [9] and at least one polymer other than a bis-propylcatechol polymer.

[12] A cured resin product, obtained by curing the resin composition according to [11].

[13] An epoxidized bis-propylcatechol represented by formula (7):

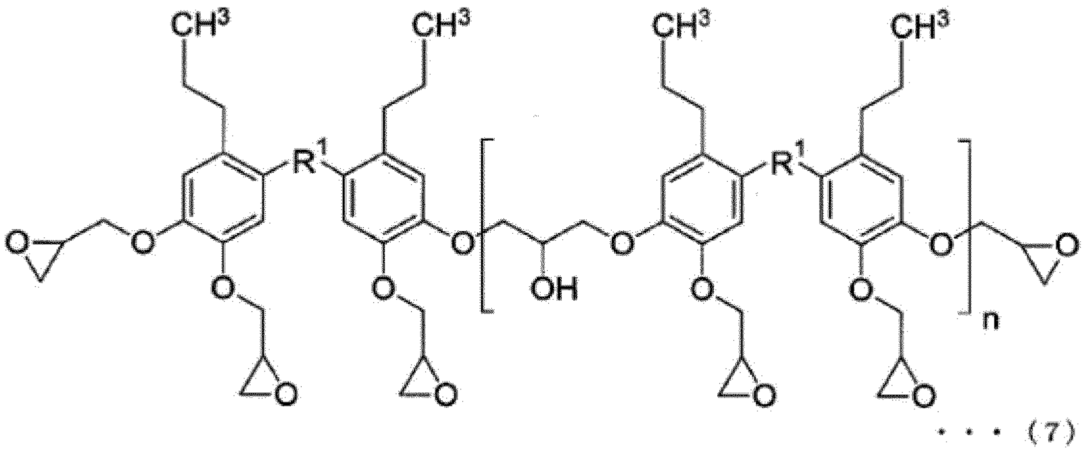

(7)

wherein $R^1$ is a divalent aliphatic hydrocarbon group having two or more carbon atoms or a divalent aromatic hydrocarbon group, and n is an integer of 0 to 300.

[14] The epoxidized bis-propylcatechol according to [13], wherein $R^1$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

[15] The epoxidized bis-propylcatechol according to [13], wherein $R^1$ is a divalent hydrocarbon group represented by $-CHR^5-$ wherein $R^5$ represents a monovalent hydrocarbon group.

[16] The epoxidized bis-propylcatechol according to [13], wherein $R^1$ is a divalent aromatic hydrocarbon group represented by formula (8):

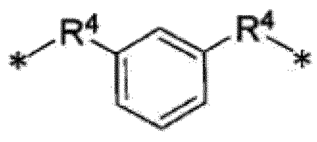

(8)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure, and * represents

a binding site to a benzene ring in the epoxidized propylcatechol structure in the above formula (7).

[17] The epoxidized bis-propylcatechol according to [13] or [15], wherein $R^1$ is a divalent hydrocarbon group represented by formula (9):

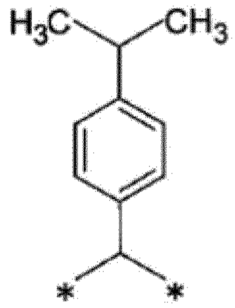

· · · (9)

wherein * represents a binding site to a benzene ring in the epoxidized propylcatechol structure in the above formula (7).

[18] The epoxidized bis-propylcatechol according to [13], wherein $R^1$ is a divalent aliphatic hydrocarbon group represented by formula (10):

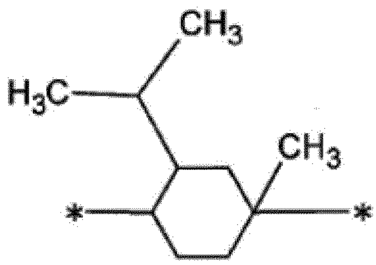

· · · (10)

wherein * represents a binding site to a benzene ring in the epoxidized propylcatechol structure in the above formula (7).

[19] The epoxidized bis-propylcatechol according to [13] or [15], wherein $R^1$ is a divalent aliphatic hydrocarbon group represented by formula (11):

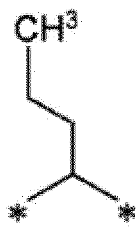

· · · (11)

wherein * represents a binding site to a benzene ring in the epoxidized propylcatechol structure in the above formula (7).

[20] The epoxidized bis-propylcatechol according to [13] or [15], wherein $R^1$ is a divalent aliphatic hydrocarbon group represented by formula (12):

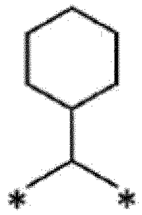

· · · (12)

wherein * represents a binding site to a benzene ring in the epoxidized propylcatechol structure in the above formula (7).

[21] A curable resin composition comprising the epoxidized bis-propylcatechol of any one of [13] to [20], and a curing agent.

[22] A method for producing the epoxidized bis-propylcatechol of any one of [13] to [20], comprising reacting the bis-propylcatechol of any one of [1] to [9] with epihalohydrin.

[23] A cured resin product, obtained by curing the curable resin composition of [21].

Advantageous Effects of Invention

The bis-propylcatechol of the present invention is a bio-based compound which is obtainable from lignocellulosic biomass and can replace the current petro-based Bisphenol A. With four reactive groups (hydroxyl group), the bis-propylcatechol of the present invention is expected to be applied to production of bio-based polymers, such as polycarbonates or polyesters, and also be starting point for a bio-based chemical platform, i.e., starting point for production of monomeric compounds such as epoxy, acrylate, methacrylate, isocyanate, cyclic carbonate or allyl compounds as well as other bio-based polymers such as epoxy polymer, polyacrylate, polymethacrylate, polyurethane or polyolefin which represent, along with polyesters and polycarbonate, worldwidely produced polymers.

Epoxidized bis-propylcatechol can be obtained from bis-propylcatechol which is a bio-based compound in the present invention. Therefore, the present invention can provide also epoxidized resins obtained from bis-propylcatechols with various linkage groups (divalent $R^1$ groups) between the phenolic moieties and be used to produce bio-based cured resin product. Such various linkage groups enable improvement of the mechanical strength or thermal properties such as glass transition temperature as compared to the epoxidized bis-propylcatechol whose phenolic moieties are linked by a methylene moiety. Finally, this present invention allows providing epoxidized cured resin products with physical properties comparable to Bisphenol-A-based materials.

DESCRIPTION OF THE EMBODIMENTS

<<Bis-propylcatechol>>

The bis-propylcatechol according to one aspect of the present invention has a structure represented by formula (1):

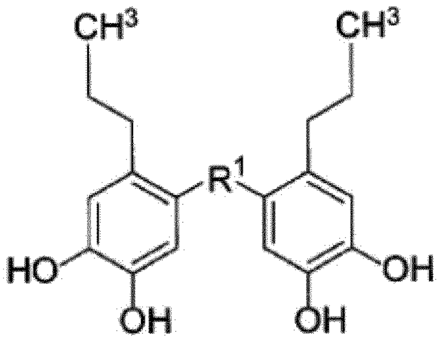

· · · (1)

wherein $R^1$ is a divalent aliphatic hydrocarbon group having two or more carbon atoms or a divalent aromatic hydrocarbon group.

In the present specification, "aromatic hydrocarbon group" means a hydrocarbon group having an aromatic ring.

In the present specification, "aliphatic hydrocarbon group" means a hydrocarbon group not having an aromatic ring.

In the formula (1), it is preferable that $R^1$ is a divalent hydrocarbon group represented by $-CR^2R^3-$ wherein each of $R^2$ and $R^3$ independently represents a monovalent hydrocarbon group. Examples of monovalent hydrocarbon groups include an alkyl group having 1 to 12 carbon atoms, a cycloalkyl group having 5 to 12 carbon atoms, a cycloalkylalkyl group having 6 to 12 carbon atoms, and an aryl group having 6 to 12 carbon atoms.

Examples of the alkyl group include a methyl group, an ethyl group, a propyl group, an isopropyl group, a butyl group, an isobutyl group, a pentyl group, a hexyl group, a heptyl group, an octyl group, a nonyl group, and a decyl group. Examples of the cycloalkyl group include a cyclopropyl group, a cyclobutyl group, a cyclopentyl group, a cyclohexyl group, cycloheptyl group, cyclooctyl group, a cyclononyl group, a cyclodecyl group, a cycloundecyl group, and a cyclododecyl group. Examples of the cycloalkylalkyl group include a cyclopropylmethyl group, a cyclobutylmethyl group, a cyclopentylmethyl group, a cyclohexylmethyl group, a cycloheptylethyl group, and a cyclooctylethyl group. Examples of the aryl group include a phenyl group, a tolyl group, an ethylphenyl group, a xylyl group, a cumenyl group, a mesityl group, o-, m- and p-methoxyphenyl groups, o-, m- and p-ethoxyphenyl group, naphthyl groups (a 1-naphthyl group and a 2-naphthyl group, etc.), and a biphenyl group.

Among those exemplified above as $R^2$ and $R^3$, a methyl group, an ethyl group, an isobutyl group, a phenyl group and the like are preferable, and a methyl group is especially preferable due to the availability and low cost of raw materials, and the ease in synthesis.

Further, $R^2$ and $R^3$ may together form a cyclic structure. For example, $R^2$ and $R^3$ may be bonded to each other such that $R^1$ has a cyclohexane structure.

Specific preferred examples of divalent aliphatic hydrocarbon group as $R^1$ include an isopropylidene group and a cycloalkylene group which may have a substituent.

As another embodiment of the invention, in the formula (1), it is preferable that $R^1$ is a divalent hydrocarbon group represented by $-CHR^5-$ wherein $R^5$ represents a monovalent hydrocarbon group. Examples of monovalent hydrocarbon groups include a monovalent aromatic hydrocarbon group and a monovalent aliphatic hydrocarbon group.

Examples of the monovalent aliphatic hydrocarbon group include a linear or branched alkyl group, a cycloalkyl group, and a cycloalkylalkyl group. Examples of the monovalent aromatic hydrocarbon group include an aryl group which may have a substituent.

Among those exemplified above as $R^5$, from the view point of excellent thermal and mechanical properties, an aryl group which may have a substituent, a linear alkyl group and a cycloalkyl group are especially preferable. Among them, from the view point of further improving the thermal properties, a cycloalkyl group and an aryl group which may have a substituent are preferable, and an aryl group which may have a substituent is particularly preferable.

The number of carbon atoms of the linear or branched alkyl group is preferably from 1 to 12, more preferably from 2 to 12, still more preferably from 3 to 12. Examples of such an alkyl group include the alkyl groups exemplified for $R^2$ and $R^3$. Among them, ethyl group, propyl group, butyl group, pentyl group, and hexyl group are preferable, propyl group, butyl group, and pentyl group are more preferable, and propyl group is particularly preferable. When $R^5$ is a propyl group, $-CHR^5-$ is represented by formula (5).

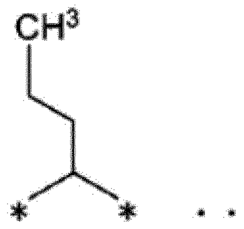

(5)

In the chemical formula, * represents a binding site to a benzene ring in the propylcatechol structure in the above formula (1). Hereinafter, the same applies to formulas (2) to (4), and formula (6).

The number of carbon atoms of the cycloalkyl group is preferably from 5 to 12, more preferably from 5 to 11, still more preferably from 5 to 10. Examples of such a cycloalkyl group include the cycloalkyl groups exemplified for $R^2$ and $R^3$. Among them, cyclopentyl group, cyclohexyl group, cycloheptyl group, cyclooctyl group, and cyclononyl group are preferable, cyclohexyl group, cycloheptyl group and cyclooctyl group are more preferable, and cyclohexyl group is particularly preferable. When $R^5$ is a cyclohexyl group, $-CHR^5-$ is represented by formula (6). The cycloalkyl group may have a substituent.

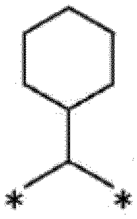

· · · (6)

The number of carbon atoms of the cycloalkylalkyl group is preferably from 4 to 12, more preferably from 5 to 11, still more preferably from 6 to 10. Examples of such a cycloalkylalkyl group include the cycloalkylalkyl groups exemplified for $R^2$ and $R^3$. Among them, cyclopropylmethyl group, cyclobutylmethyl group, cyclopentylmethyl group, cyclohexylmethyl group, and cycloheptylethyl group are preferable, cyclohexylmethyl group, cyclopentylmethyl group, and cyclobutylmethyl group are more preferable, and cyclohexylmethyl group is particularly preferable. The cycloalkylalkyl group may have a substituent.

The number of carbon atoms of the aryl group is preferably from 6 to 12, more preferably from 6 to 11, still more preferably from 6 to 10. Examples of such a aryl group include the aryl groups exemplified for $R^2$ and $R^3$. Among them, phenyl group, tolyl group, ethylphenyl group, xylyl group, and cumenyl group are preferable, phenyl group, tolyl group, and xylyl group are more preferable, and phenyl group is particularly preferable. The aryl group may have a substituent. The aryl group may have a substituent (the tolyl group, the ethylphenyl group, the xylyl group, and the cumenyl group are aryl groups having a substituent). The number of the substituents depends on the carbon number of the aryl group, but is preferably from 1 to 10, more preferably from 1 to 9, still more preferably from 1 to 8.

The substituent is preferably a linear or branched alkyl group. The number of carbon atoms of the alkyl group is preferably 1 to 10, more preferably 1 to 9, still more preferably 1 to 8. Examples of such an alkyl group include a methyl group, an ethyl group, a propyl group, an iso-propyl group, a butyl group, an iso-butyl group, a tert-butyl group, pentyl group, hexyl group, and the like. Among them, the iso-propyl group, the iso-butyl group, the tert-butyl group, the pentyl group, and the hexyl group are preferable, the iso-propyl group, the iso-butyl group, and the tert-butyl group are more preferable, and the iso-propyl group is particularly preferable. Accordingly, as $R^5$, it is preferable that the aryl group is the phenyl group, the number of substituents is 1 to 5, and the substituent is at least one alkyl group selected from the group consisting of the iso-propyl group, the iso-butyl group, and the tert-butyl group. Among these, as $R^5$, it is most preferable that the aryl group is the phenyl group, the number of substituents is 1, and the substituent is the iso-propyl group. Such $-CHR^5-$ is represented by the formula (3).

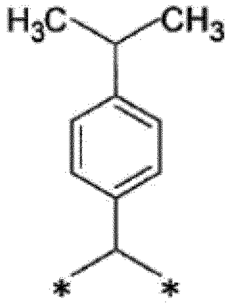

· · · (3)

As another embodiment of the invention, in the formula (1), it is preferable that $R^1$ is a divalent aromatic hydrocarbon group represented by formula (2), and a divalent aliphatic hydrocarbon group having a cyclic structure represented by formula (4).

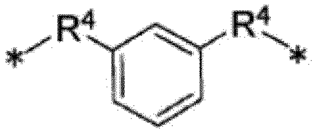

· · · (2)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

Examples of $R^2$ and $R^3$ are the same as those in the case where $R^1$ in the formula (1) is a divalent hydrocarbon group represented by $-CR^2R^3-$.

In the formula (2), it is particularly preferable that each of $R^2$ and $R^3$ independently represents a methyl group to increase the mechanical and thermal properties of materials.

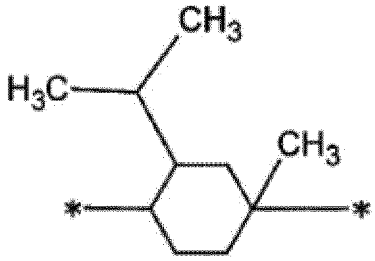

· · · (4)

The bis-propylcatechol of the present invention can be used as a substitute for BPA in production of various polymers such as polycarbonates or polyesters. For example, polycarbonate is currently produced from BPA and phosgene ($COCl_2$). Polycarbonate can be produced by reacting the bis-propylcatechol with phosgene ($COCl_2$), following substantially the same method as conventionally employed for producing polycarbonate from BPA and phosgene. Alternatively, polycarbonate can be produced by transesterification reaction between the bis-propylcatechol and a dialkyl or diaryl carbonate such as dimethylcarbonate or diphenyl carbonate. This transesterification reaction can also be performed following substantially the same method as conventional employed for producing polycarbonate from BPA a dialkyl or diaryl carbonate.

<<Method for Producing Bis-propylcatechol>>

As regards the method for synthesizing the bis-propylcatechol according to the present invention, there is no particular limitation. For example, the bis-propylcatechol can be synthesized substantially following the method as described in Non-Patent Document 3 except that formaldehyde is not used. Specifically, the bis-propylcatechol can be produced by a method including reacting a propylcatechol with a reactant suitable for forming a desired $R^1$ group. For example, the reactant may be a ketone such as acetone, or cyclohexanone when $R^1$ group is the aforementioned $-CR^2R^3-$; an alcohol such as 1,4-bis(2-hydroxyisopropyl)benzene when $R^1$ group is the aforementioned divalent aromatic hydrocarbon group represented by formula (2). The reactant may be an aldehyde represented by $R^5$-CHO when $R^1$ group is the aforementioned $-CHR^5-$. Here, $R^5$ is the same as $R^5$ in $-CHR^5-$ explained above. Specifically, the reactant is cuminaldehyde when $R^1$ group is the aromatic hydrocarbon group represented by the above formula (3); butylaldehyde when $R^1$ group is the alkyl group represented by the above formula (5); and cyclohexaecarboxaldehyde when $R^1$ group is the cycloalkyl group represented by the above formula (6). Further, when $R^1$ group is the aforementioned divalent aliphatic hydrocarbon group having a cyclic structure represented by the above formula (4), the reactant may be a monoterpene such as 1-isopropyl-4-methyl-1,4-cyclohexadiene.

The molar ratio (propylcatechol / the reactant) of propylcatechol and a reactant suitable for forming a desired $R^1$ group for the reaction is usually 2 to 2.5.

The propylcatechol can be obtained from lignocellulosic biomass. For details of the method for obtaining propylcatechol from lignocellulosic biomass, reference can be made to, for example, Zhuohua Sun et al., "Bright Side of Lignin. Depolymerization: Toward New Platform Chemicals", Chemical Reviews 118:2, 614-678, 2018.

The reaction may be carried out in the presence of a catalyst if necessary. As the catalyst, for example, those conventionally used for producing bisphenol compounds can be used. Specifically, the catalyst may be strongly acidic solution such as hydrochloric acid, hydrobromic acid, phosphoric acid, and sulfuric acid, or strongly acidic cation exchange resins such as sulfonic acid type, preferably those partially neutralized with a sulfur-containing amine compound. As the sulfur-containing amine compound, ordinary promoters used for the synthesis of Bisphenol A such as 2-(4-pyridyl)ethanethiol, 2-mercaptoethylamine, 3-mercaptopropylamine, N,N-dimethyl-3-mercaptopropylamine, N,N-di-n-butyl-4-mercaptobutylamine, and 2,2-dimethylthiazolidine can be used. Such a promoter is used in an amount of usually 2 to 30 mol %, preferably 5 to 20 mol % based on the acid group in the acid solution or acid ion exchanger.

The reaction conditions may also be those conventionally used for producing bisphenol compounds. Specifically, the reaction temperature for this reaction is preferably 0 to 200 °C, and more preferably 25 to 150°C. As for the reaction pressure, this reaction can be performed under atmospheric pressure. Further, the reaction time is preferably 30 minutes to 48 hours, and more preferably 2 to 24 hours.

<<Resin Composition Containing Bis-propylcatechol>>

The resin composition (also referred to as "composition (A)") according to another aspect of the present invention

includes the aforementioned bis-propylcatechol and at least one polymer other than a bis-propylcatechol polymer. In the present specification, "bis-propylcatechol polymer" means a polymer synthesized using bis-propylcatechol as a raw material. In this composition (A), the bis-propylcatechol functions as a crosslinking agent so as to improve various physical and mechanical properties of the polymer of interest.

Examples of the polymer include, but are not limited to, one or more of the following: polystyrene, polysulfone, polymethyl methacrylate, polyacrylonitrile, polybutylacrylate, polymethylmethacrylate, polybutadiene, polyoxymethylene (acetal), high impact polystyrene, polyamide, polybutylene terephthalate, polycarbonate, polyethylene, polyethylene terephthalate, polyetheretherketone, polyetherimide, polyethersulfone, polyphthalamide, polyphenylene ether, polyphenylene sulfide, polyurethane, polyester, and poly(styrene-acrylonitrile) as well of mixtures of any of the foregoing. The polymer employed in the composition can have any suitable molecular weight depending on the purpose and the like. In certain embodiments, the polymer present in the resin composition can have a molecular weight of 344 to 20000 $g.mol^{-1}$, more preferably 400 to 8000 $g.mol^{-1}$.

In the present specification, "weight average molecular weight" is a molecular weight in terms of polystyrene obtained by gel permeation chromatography (GPC) measurement.

The amount of the bis-propylcatechol in the composition (A) is not particularly limited and may be appropriately adjusted in view of the type of the polymer, the purpose of the resin composition, etc. For example, the amount of the bis-propylcatechol in the composition (A) is preferably in the range of 1 to 90 parts by mass, more preferably 2 to 50 parts by mass relative to 100 parts by mass of the composition (A).

The composition (A) may further include any known additives. Examples of additives which may be employed include, but are not limited to, one or more of the following: ultraviolet light stabilizers, heat stabilizers, antioxidants, colorants (for example, pigments or dyes), antistatic agents, flame retardant agents, smoke suppressants, foaming agents, electrical conductivity agents, lubricants and abrasion resistant. The amount of the additive in the composition (A) is preferably in the range of 0.1 to 5 and more preferably 0.3 to 3 by mass relative to 100 parts by mass of the composition (A).

<<Cured Resin Product>>

The cured resin product according still another aspect of the present invention is one obtained by curing the aforementioned composition (A). Curing can be done by any appropriate method depending on the type of the polymer used in the composition (A). For example, curing can be done by heat, ultraviolet radiation, etc. If necessary, an appropriate curing agent may be used.

«Epoxidized Bis-Propylcatechol»

The epoxidized bis-propylcatechol according to still another aspect of the present invention is represented by formula (7):

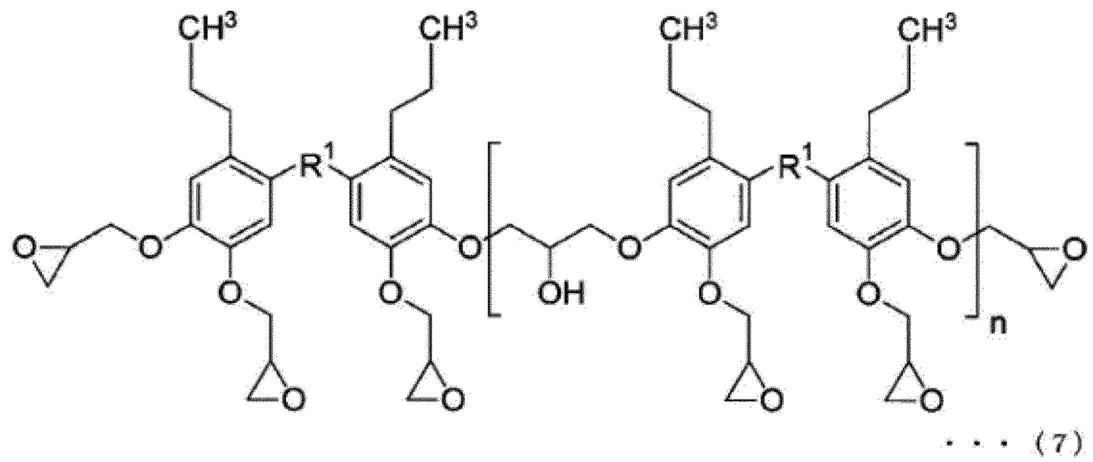

・・・(7)

wherein $R^1$ is a divalent aliphatic hydrocarbon group having two or more carbon atoms or a divalent aromatic hydrocarbon group, and n is an integer of 0 to 300.

In the formula (7), n is 0 to 300 and preferably 1 to 150. When n is in this range, the epoxidized bis-propylcatechol exhibits excellent adhesion strength to other material as well as moderate viscosity suitable for handling.

In the formula (7), $R^1$ is as defined for the formula (1). Specifically, it is preferable that $R^1$ is a divalent hydrocarbon

group represented by $-CR^2R^3-$ wherein each of $R^2$ and $R^3$ independently represents a monovalent hydrocarbon group. Examples of $R^2$ and $R^3$ are the same as those in the case where $R^1$ in the formula (1) is a divalent hydrocarbon group represented by $-CR^2R^3-$.

Further, $R^2$ and $R^3$ may together form a cyclic structure. For example, $R^2$ and $R^3$ may be bonded to each other such that $R^1$ has a cyclohexane structure.

As another embodiment of the invention, in the formula (7), it is preferable that $R^1$ is a divalent hydrocarbon group represented by $-CHR^5-$ wherein $R^5$ represents a monovalent hydrocarbon group. Examples of $R^5$ are the same as those in the case where $R^1$ in the formula (1) is a divalent hydrocarbon group represented by $-CHR^5-$.

Specifically, in the formula (7), it is also most preferable that $R^1$ is a divalent hydrocarbon group represented by formula (9) and a divalent aliphatic hydrocarbon group represented by formula (11) or formula (12).

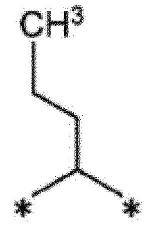

・・・(11)

In the chemical formula, * represents a binding site to a benzene ring in the epoxidized propylcatechol structure in the above formula (7). Hereinafter, the same applies to formulas (8) to (10), and formula (12).

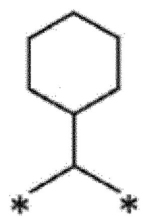

・・・(12)

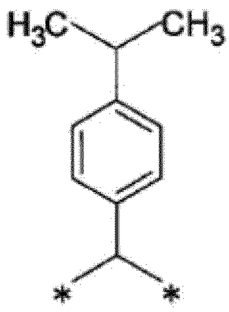

・・・(9)

As another embodiment of the invention, in the formula (7), it is preferable that $R^1$ is a divalent aromatic hydrocarbon group represented by formula (8), and a divalent aliphatic hydrocarbon group having a cyclic structure represented by formula (10).

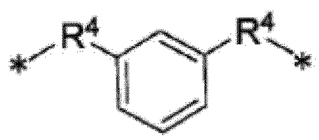

・・・(8)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

Examples of $R^2$ and $R^3$ are the same as those in the case where $R^1$ in the formula (1) is a divalent hydrocarbon group represented by $-CR^2R^3-$. In the formula (8), it is particularly preferable that each of $R^2$ and $R^3$ represents a methyl group to increase mechanical and thermal properties of cured resin using epoxidized bis-propylcatechol of the present

embodiment.

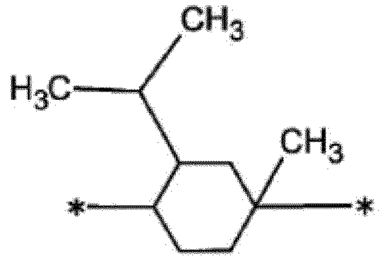

· · · (10)

<<Method for Producing Epoxidized Bis-Propylcatechol>>

The epoxidized bis-propylcatechol can be produced by a method including reacting the aforementioned bis-propylcatechol with epihalohydrin.

Examples of the epihalohydrin include, but are not limited to, one or more of the following: epichlorohydrin, epibromohydrin, epiiodohydrin, epifluorohydrin and any mixture of these epihalohydrins. Among these, epichlorohydrin is preferable.

It is preferable that epihalohydrin is used in an amount of 2.2 to 100 mol relative to 1 mole of the bis-propylcatechol compound.

The reaction to produce epoxidized bis-propylcatechol is carried out, but are not limited to, in the presence of a catalyst and an alkali compound. Examples of the catalyst include quaternary ammonium salts and the like, such as benzyltriethylammonium chloride, benzyltriethylammonium bromide, tetra-n-butylammonium fluoride, and tetra-n-butylammonium bromide. The amount of the catalyst used is prefably 0.01 to 1 mol and more preferably 0.02 to 0.2 mol relative to 1 mol of the bis-propylcatechol compound.

Examples of the alkali compound include alkali metal hydroxides such as sodium hydroxide and potassium hydroxide, and alkali metal carbonates such as sodium carbonate and potassium carbonate, and the amount of the alkali compound used is preferably 1.5 to 8 mol and more preferably 2 to 4 mol relative to 1 mol of the bis-propylcatechol compound.

Regarding the specific procedure and conditions for this reaction, the same as those for producing conventional epoxy resins using bisphenol A can be employed. For example, the reaction temperature for this reaction is preferably -20 to 200°C, and more preferably 0 to 150°C. As for the reaction pressure, this reaction can be performed under atmospheric pressure. Further, the reaction time is preferably 30 minutes to 48 hours, and more preferably 1 to 24 hours.

<<Curable Resin Composition>>

The curable resin composition (also referred to as "composition (B)") according to still another aspect of the present invention, includes the aforementioned epoxidized bis-propylcatechol, and a curing agent. The curing agent may be any of those generally used for conventional epoxy resins produced with Bisphenol A. Examples of the curing agent include, but are not limited to, one or more of the following: diethylenetriamine, triethylenetetramine, isophoronediamine, diaminodiphenylmethane, diaminodiphenylsulfone, polyamides, dicyandiamide, hexahydrophthalic anhydride, methyltetrahydrophthalic anhydride, methyl nadic anhydride, tertiary amines, imidazoles, and amine complexes of boron trifluoride.

Mixing of the epoxidized bis-propylcatechol and the curing agent to form the composition (B) can be in any order and by any appropriate means known in the art for two component epoxy compositions.

The amount of epoxidized bis-propylcatechol is preferably 1 to 99 parts by mass, more preferably 2 to 95 parts by mass, and still more preferably 3 to 90 parts by mass relative to 100 parts by mass of the composition (B).

The amount of the curing agent in the composition (B) is preferably in the range of 1 to 90 parts by mass, more preferably 1 to 50, and still more preferably 1 to 30 by mass relative to 100 parts by mass of the composition (B). When the amount of curing agent is not less than the above lower limit, a sufficient curing rate can be achieved. When the amount of curing agent is not more than the above upper limit, deterioration of thermal and mechanical properties can be prevented.

When an amine compound is used as a curing agent, the mixing can be carried out by any suitable means known in the art. It is preferable to adjust the molar ratio of the epoxy group in the epoxidized bis-propylcatechol to the hydrogen (active hydrogen) bonded to the N atom in the amine compound to be equivalent.

The composition (B) may further include any known additives. Examples of additives which may be employed include, but are not limited to, those mentioned above in connection with the composition (A). The amount of the additive

in the composition (B) is preferably in the range of 0.1 to 5 and more preferably 0.3 to 3 by mass relative to 100 parts by mass of the composition (B).

<<Cured Resin Product>>

The cured resin product according to still another aspect of the present invention is one obtained by curing the aforementioned composition (B). The conditions for curing can be appropriately adjusted depending on the application of the cured resin. For example, curing may be carried out preferably at a temperature ranging from 15°C to 200°C, and more preferably 50°C to 190°C.

<<Analysis>>

The glass transition temperature (Tg) of cured resin product may also be measured by DSC (Differential scanning calorimetry).
The transition relaxation temperature ($T\alpha$) of cured resin product may also be obtained by DMA (Dynamic mechanical analysis).
Hardness Shore D of cured resin product may also be measured with a durometer.

[Examples]

Hereinbelow, the present invention will be described with reference to Examples which, however, should not be construed as limiting the scope of the present invention.

<Materials>

2-methoxy-4-propylphenol, sodium hydroxide (NaOH), tetrabutylammonium bromide (TBABr), isophorone diamine (IPDA), hydrobromic acid (48%) (HBr), sulfuric acid (98%) ($H_2SO_4$), and methanol were purchased from Sigma Aldrich Company. Cuminaldehyde, butyraldehyde and cyclohexanecarboxaldehyde were bought from Tokyo Chemical Industry Co., Ltd. (TCI). Epichlorohydrin, anhydrous magnesium sulfate ($MgSO_4$), tetrahydrofuran and ethyl acetate were bought from Wako Company. Diglycidyl ether bisphenol A (DGEBA) was obtained from Mitsubishi Chemical Company. Deuterated solvent (DMSO-$d_6$) was purchased from Cambridge Isotope Laboratory Inc.

<Characterization methods>

[NMR]

In the following Examples and Comparative Examples, chemical structures of the molecules were determined by $^1$H and $^{13}$C, NMR-spectroscopy using a Jeol 400 MHz spectrometer equipped with a Royal probe at room temperature. External reference was tetramethylsilane (TMS). Shifts are given in ppm. NMR samples were prepared as follows: around 10 mg of product for $^1$H and $^{13}$C experiment in around 0.75 mL of DMSO-$d_6$.
In the following Examples and Comparative Examples, the amount of substance (in grams) containing one gram-equivalent of epoxy functions called the epoxy equivalent weight (EEW), was determined by $^1$H NMR spectroscopy titration. First, a solution of DMSO-$d_6$ containing toluene as internal standard was prepared (c=6.90 g.L$^{-1}$). Then a known amount of epoxidized bis-propylcatechol (around 60 mg) and the toluene containing deuterated solution (around 800 mg) were weighed and transferred into an NMR tube. One characteristic epoxy peak at 2.70 ppm was chosen. The EEW was calculated from Equation (1) by comparing the integral ($\int H_{toluene}$) of the protons bonded to toluene with the integral ($\int_{Hepoxy}$) of epoxy protons.
In formula (1), $m_{epoxydized\ bis\text{-}propylcatechol}$ is the mass of epoxidized bis-propylcatechol, $m_{toluene}$ is the mass of toluene, and $M_{toluene}$ is the molecular weight of toluene.

$$EEW = \left(\frac{\int_{7.09}^{7.32} H_{toluene}}{\int_{2.64}^{2.75} H_{epoxy}}\right)\left(\frac{m_{epoxydized\ bis-propylcatechol}}{m_{toluene}}\right) M_{toluene} \quad (1)$$

[GPC Measurement]

In the following Examples and Comparative Examples, the molecular weight of chemicals were measured by using a GPC system manufactured by Shimadzu Corporation with columns "KF803L"x2 manufactured by Showa Denko K.K. (exlusion limit molecular weight: 70,000) was used. The column temperature was set at 40°C., and the flow rate was set at 1.0 $ml.min^{-1}$. Tetrahydrofuran was used as an eluent, and a RI was used as a detector. In the method for calculating a molecular weight, a calibration curve was made using standard polystyrenes "TSK standard POLYSTYRENE" having weight average molecular weights (Mw) of 96,400; 37,900; 18,100; 9,100; 5,970; 2,630; 1,050 and 500, respectively. The weight average molecular weight (Mw), the number average molecular weight (Mn) and the molecular weight distribution (Mw/Mn) were obtained by calculation.

[Thermal stability Measurement]

In the following Examples and Comparative Examples, glass transition temperatures of the materials were determined by differential scanning calorimetry using scanning calorimetry (DSC) using a Shimadzu DSC-60 calorimeter. Argon was used as the inert gas. Samples were placed in aluminum pans and the thermal properties were recorded at 10 $°C.min^{-1}$.

[Thermomechanical Measurement]

In the following Examples and Comparative Examples, thermomechanical properties of the materials were determined by dynamic mechanical analyses (DMA) using a SII Nanotechnology DMS6100. Uniaxial stretching of samples (10 x 4 x $1mm^3$) was performed while heating at a rate of $2°C.min^{-1}$ from 0°C to 250°C, keeping frequency at 1 Hz. $T\alpha$ was determined by the temperature, in °C, of transition from vitreous to elastic domain of material determined at the maximum of the tan $\delta$ curve.

In the following Examples and Comparative Examples, Shore D hardness was measured on a Shore durometer GS-720G from Teclock. Samples with dimensions 0.5 cm thickness were prepared for the measurement. The measurement was performed at five different places in this sample, and their average value was determined.

Example 1:

• Synthesis of propylcatechol

Propylcatechol was obtained following the method described in Non-Patent Document 2 and the reaction scheme shown in formula (13) below.

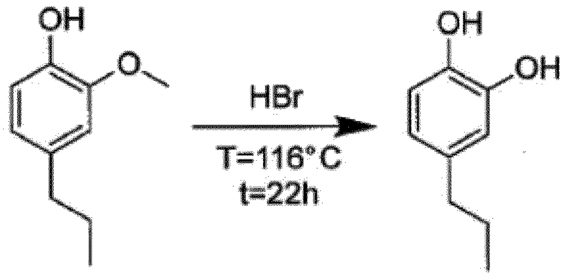


2-methoxy-4-propylphenol Propylcatechol ・・・ (13)

Briefly, 2-methoxy-4-propylphenol (100 g, 601.61 mmol) was added to 48% aqueous hydrobromic acid solution (250 g, 3.09 mol). The reaction mixture was magnetically stirred at 120 °C for 15.5 h and cooled to ambient temperature. Then deionized water was added to the solution and the product was extracted three times with ethyl acetate (3x500 mL). The organic layer were combined, washed twice with deionized water, dried over anhydrous $MgSO_4$ and concentrated under reduced pressure. The product was obtained quantitatively as brown liquid with a 91% yield. The $^1H$ and $^{13}C$ NMR spectra of the resulting product in deuterated dimethyl sulfoxide were a follow.

$^1H$ NMR (400 MHz, DMSO-$d_6$) $\delta$(ppm) = 8.65 (s, 1H), 8.55(s, 1H), 6.39-6.62(m, 3H), 2.36 (t, 2H), 1.50(sex, 2H), 0.85(t, 3H).

$^{13}C$ NMR (100 MHz, DMSO-$d_6$) $\delta$(ppm) = 144.93, 143.08, 132.94, 118.88, 115.71, 115.38, 36.71, 24.33, 13.65.

• Synthesis of bis-propylcatechol 1

Bis-propylcatechol 1 was obtained following the reaction scheme shown in formula (14) below.

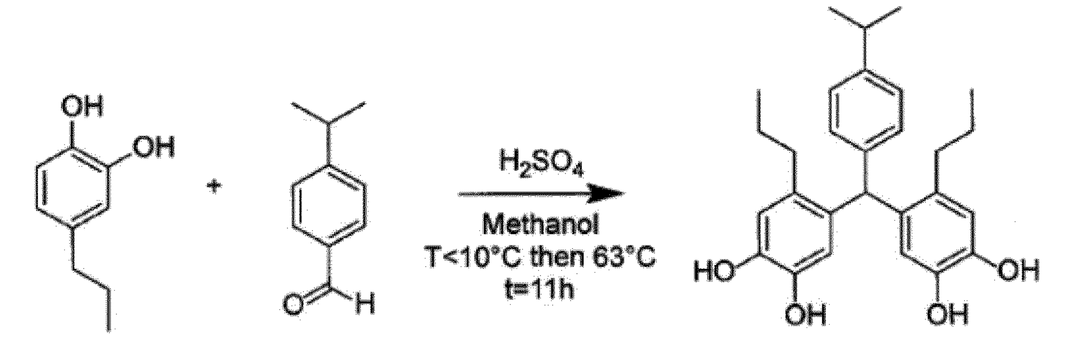


Propylcatechol Cuminaldehyde Bis-propylcatechol 1

・・・(14)

Briefly, cuminaldehyde (9.69 g, 65.38 mmol) and propylcatechol obtained in the above (20.00 g, 135.45 mmol) were dissolved in 20 mL of methanol in a 200 mL round-bottom flask at 5°C. An aqueous $H_2SO_4$ solution (3.22 g (36.69 mmol) of 98% $H_2SO_4$, previously diluted in 5 mL of methanol) was added dropwise to the reaction mixture under stirring. Then the flask was sealed, and the reaction mixture was further stirred for 1 h at a temperature below to 10°C then during 10 hours at 63°C. Then, the reaction mixture was neutralized with saturated aqueous $NaHCO_3$ and the product was extracted with diethyl ether (3×100 mL), and the combined organic fractions were washed with deionized water, dried with anhydrous $MgSO_4$, filtered and concentrated under reduced pressure. The product was obtained quantitatively as brown solid with a 97% yield. The $^1$H and $^{13}$C NMR spectra of the resulting product in deuterated dimethyl sulfoxide were a follow.
$^1$H NMR (400 MHz, DMSO-$d_6$) δ(ppm) = 8.51 (s, 2H), 8.50 (s, 2H), 7.14-6.85 (dd, 4H), 6.52 (s, 2H), 6.17 (s, 2H), 5.47(s, 1H), 2.84 (sep, 1H), 2.25 (t, 4H), 1.37 (sex, 4H), 1.18 (d, 6H), 0.81 (t, 6H).
$^{13}$C NMR (100 MHz, DMSO-$d_6$) δ(ppm) = 145.47, 143.04, 142.42, 142.36, 132.59, 130.53, 129.00, 125.94, 117.18, 116.78, 47.01, 33.66, 32.88, 23.91, 23.74, 14.04.

• Synthesis of epoxidized bis-propylcatechol 1 resin

Epoxidized bis-propylcatechol 1 resin was obtained following the reaction scheme shown in formula (15) below.

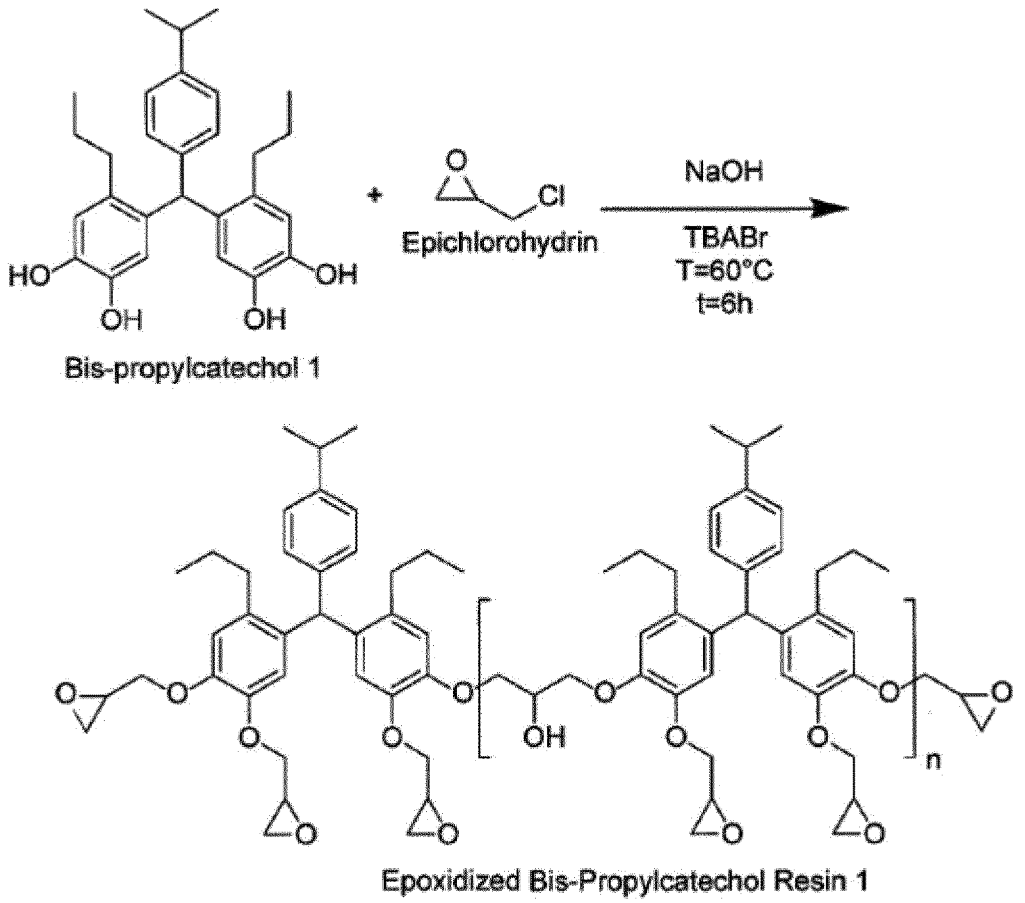


Bis-propylcatechol 1

Epichlorohydrin

Epoxidized Bis-Propylcatechol Resin 1

・・・(15)

Briefly, bis-propylcatechol 1 obtained in the above(20.00 g, 46.0 mmol, 1.00 eq.), TBABr (1.48 g, 4.60 mmol, 0.10 eq.) and epichlorohydrin (85.16 g, 920.43 mmol, 20 eq.) were added in a four-neck round bottom flask equipped with a magnetic stirred and a condenser. The reaction was conducted at 60°C during 3 hours. Then an aqueous solution of NaOH (50w%, 4 eq) was added. The reaction was still conducted during 3 hours at 60°C. Then, deionized water was added to the mixture to dilute four times the NaOH solution and an equal volume of ethyl acetate was added. The mixture was stirred and the aqueous phase was extracted 2 more times with ethyl acetate (2x300 mL). Organic phases were combined, rinsed with brine and dried on anhydrous $MgSO_4$. Ethyl acetate and excess of epichlorohydrin were removed under vacuum. The epoxidized bis-propylcatechol resin was obtained quantitatively as orange solid with a 90% yield. The EEW of epoxidized bis-propylcatechol 1 was titrated by $^1$HNMR at 187 g.eq$^{-1}$. The measurement by the GPC method of the molecular weight (in terms of standard polystyrene) of the resulting epoxidized bis-propylcatchol 1 resin resulted in a number average molecular weight Mn of 759 g.mol$^{-1}$, a weight average molecular weight Mw of 766 g.mol$^{-1}$, and a molecular weight distribution Mw/Mn of 1.01. Accordingly, the average value of n calculated from Mn was 1.15.

The $^1$H and $^{13}$C NMR spectra of the resulting product in deuterated dimethyl sulfoxide were a follow.
$^1$H NMR (400 MHz, DMSO-$d_6$) $\delta$(ppm) = 7.14-6.92 (dd, 4H), 6.67-6.12 (m, 4H), 5.63(s, 1H), 5.00 (m, 0.60H) 4.29-4.00 (m, 4H), 3.83-3.59 (m, 4H), 3.15 (m, 1.5H), 2.82 (m, 2H), 2.70 (m, 2H), 2.38 (t, 3.70H), 1.41 (sex, 4H), 1.17 (d, 6H), 0.82 (t, 6H).
$^{13}$C NMR (100 MHz, DMSO-$d_6$) $\delta$(ppm) = 147.89, 146.58, 141.31, 133.79, 129.01, 126.15, 116.96, 115.62, 69.89, 49.95, 47.31, 43.75, 33.90, 32.96, 23.96, 23.67, 14.00.

Example 2:

- Synthesis of bis-propylcatechol 2

Bis-propylcatechol 2 was obtained following the reaction scheme shown in formula (16) below.

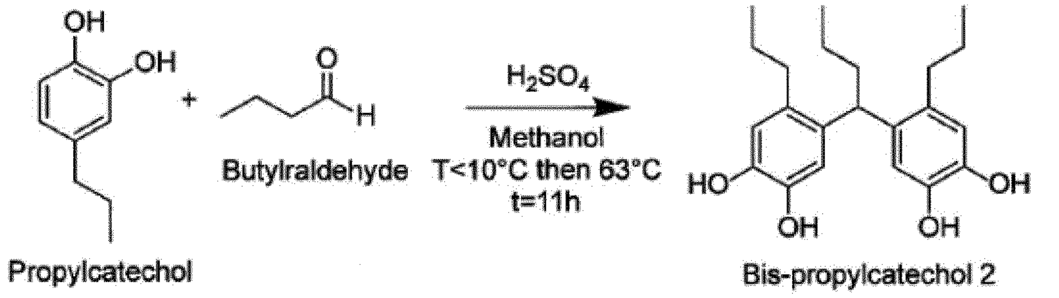


・・・(16)

Briefly, butylraldehyde (2.36 g, 32.69 mmol) and propylcatechol obtained in the above (10.00 g, 65.71 mmol) were dissolved in 8 mL of methanol in a 100 mL round-bottom flask at 0°C. An aqueous $H_2SO_4$ solution (1.61 g (16.3 mmol) of 98% $H_2SO_4$, previously diluted in 2 mL of methanol) was added dropwise to the reaction mixture under stirring. Then the flask was sealed, and the reaction mixture was further stirred for 1 h at a temperature below to 10°C then during 10 hours at 63°C. Then, the reaction mixture was neutralized with saturated aqueous $NaHCO_3$ and the product was extracted with diethyl ether (3×200 mL), and the combined organic fractions were washed with deionized water, dried with anhydrous $MgSO_4$, filtered and concentrated under reduced pressure. The product was obtained quantitatively as brown solid with a 82% yield. The $^1H$ and $^{13}C$ NMR spectra of the resulting product in deuterated dimethyl sulfoxide were a follow.

$^1H$ NMR (400 MHz, DMSO-$d_6$) δ(ppm) = 8.53 (s, 2H), 8.48 (s, 2H), 6.67-6.22 (m, 4H), 4.37 (t, 1H), 2.22 (t, 4H), 1.68 (sex, 2H), 1.32 (sex, 4H), 1.13 (sex, 2H), 0.89 (t, 6H), 0.79 (t, 3H).

$^{13}C$ NMR (100 MHz, DMSO-$d_6$) δ(ppm) = 143.99, 143.35, 121.03, 115.71, 115.36, 39.84, 37.67, 34.05, 24.77, 21.23, 14.34, 13.88.

• Synthesis of epoxidized bis-propylcatechol resin 2

Epoxidized bis-propylcatechol resin 2 was obtained following the reaction scheme shown in formula (17) below.

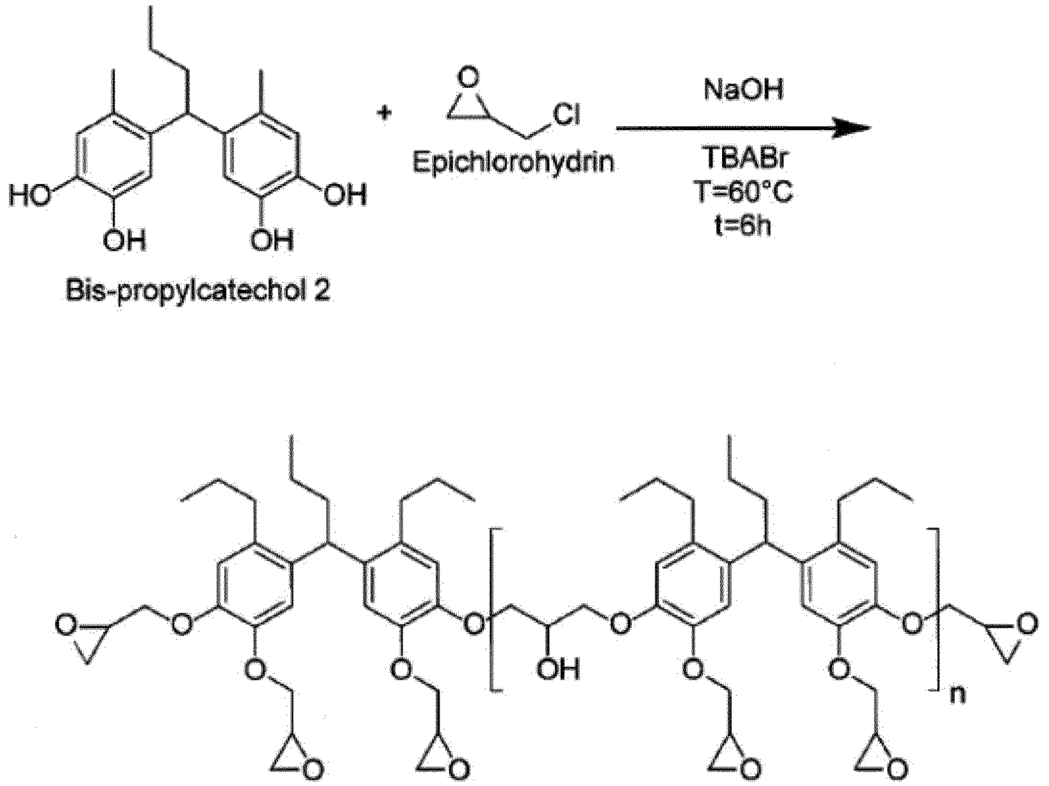


Bis-propylcatechol 2

Epoxidized Bis-Propylcatechol Resin 2

• • • (17)

Briefly, bis-propylcatechol 2 obtained in the above(10.82g, 30.17 mmol, 1.00 eq.), TBABr (0.97 g, 3.02 mmol, 0.10 eq.) and epichlorohydrin (57.64 g, 623.00 mmol, 20 eq.) were added in a four-neck round bottom flask equipped with a magnetic stirred and a condenser. The reaction was conducted at 60°C during 3 hours. Then an aqueous solution of NaOH (50w%, 4 eq) was added. The reaction was still conducted during 3 hours at 60°C. Then, deionized water was added to the mixture to dilute four times the NaOH solution and an equal volume of ethyl acetate was added. The mixture was stirred and the aqueous phase was extracted 2 more times with ethyl acetate (2x200 mL). Organic phases were combined, rinsed with brine and dried on anhydrous $MgSO_4$. Ethyl acetate and excess of epichlorohydrin were removed under vacuum. The epoxidized bis-propylcatechol resin 2 was obtained quantitatively as orange solid with a 80% yield. The EEW of epoxidized bis-propylcatechol resin 2 was titrated by $^1H$ NMR at 210 $g.eq^{-1}$. The measurement by the GPC method of the molecular weight (in terms of standard polystyrene) of the resulting epoxidized bis-propylcatchol resin 2 resulted in a number average molecular weight Mn of 712 $g.mol^{-1}$, a weight average molecular weight Mw of 720 $g.mol^{-1}$, and a molecular weight distribution Mw/Mn of 1.01. Accordingly, the average value of n calculated from Mn was 1.22.

The $^1H$ and $^{13}C$ NMR spectra of the resulting product in deuterated dimethyl sulfoxide were a follow.

$^1H$ NMR (400 MHz, DMSO-$d_6$) δ(ppm) = 6.89-6.64 (m, 4H), 5.04 (t, 1H), 4.32 (m, 4H), 3.84 (m, 4H), 3.31 (m, 4H), 2.82 (m, 4H), 2.69 (m, 4H), 2.51 (t, 4H), 1.70 (sex, 2H), 1.47 (sex, 4H), 1.22 (sex, 2H), 0.89 (t, 6H), 0.79 (t, 3H).

$^{13}C$ NMR (100 MHz, DMSO-$d_6$) δ(ppm) = 147.02, 146.64, 137.10, 136.82, 122.01, 115.96, 70.84, 50.62, 45.05, 40.96, 37.80, 34.37, 24.89, 21.54, 14.45, 14.02.

Example 3:

• Synthesis of bis-propylcatechol 3

Bis-propylcatechol 3 was obtained following the reaction scheme shown in formula (18) below.

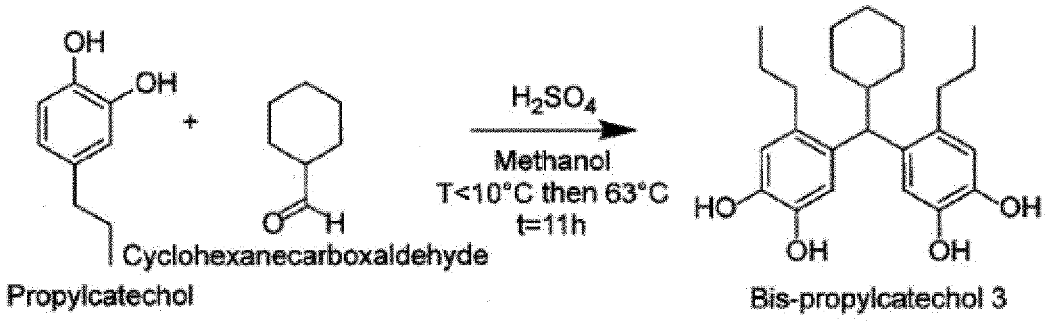


・・・(18)

Briefly, cyclohexanecarboxaldehyde (3.67 g, 32.69 mmol) and propylcatechol obtained in the above (10.00 g, 65.71 mmol) were dissolved in 8 mL of methanol in a 100 mL round-bottom flask at 0°C. An aqueous $H_2SO_4$ solution (1.61 g (16.34 mmol) of 98% $H_2SO_4$, previously diluted in 2.0 mL of methanol) was added dropwise to the reaction mixture under stirring. Then the flask was sealed, and the reaction mixture was further stirred for 1 h at a temperature below to 10°C then during 10 hours at 63°C. Then, the reaction mixture was neutralized with saturated aqueous $NaHCO_3$ and the product was extracted with diethyl ether (3×200 mL), and the combined organic fractions were washed with deionized water, dried with anhydrous $MgSO_4$, filtered and concentrated under reduced pressure. The product was obtained quantitatively as brown solid with a 87% yield. The $^1$H and $^{13}$C NMR spectra of the resulting product in deuterated dimethyl sulfoxide were a follow.

$^1$H NMR (400 MHz, DMSO-*d*$_6$) δ(ppm) = 8.51 (s, 2H), 8.48 (s, 2H), 6.85-6.35 (m, 4H), 4.98 (d, 1H), 2.48-2.27(m, 4H), 1.44 (sex, 4H), 0.95 (t, 6H), 1.70-0.74 (m, 11H).

$^{13}$C NMR (100 MHz, DMSO-*d*$_6$) δ(ppm) = 142.87, 142.62, 132.46, 130.85, 115.56, 114.97, 45.09, 37.06, 33.33, 31.58, 26.33, 24.58, 24.05, 14.28.

• Synthesis of epoxidized bis-propylcatechol resin 3

Epoxidized bis-propylcatechol resin 3 was obtained following the reaction scheme shown in formula (19) below.

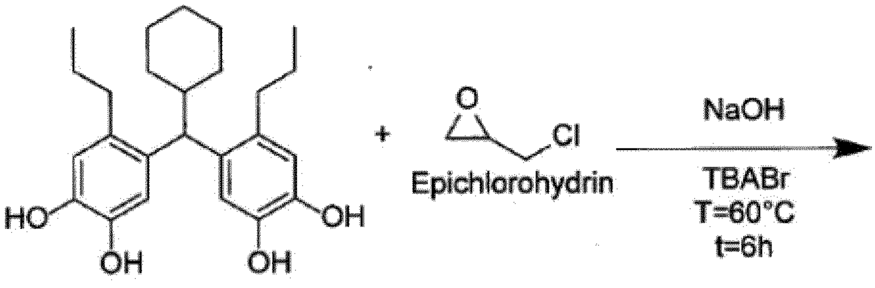


Bis-propylcatechol 3

Epichlorohydrin

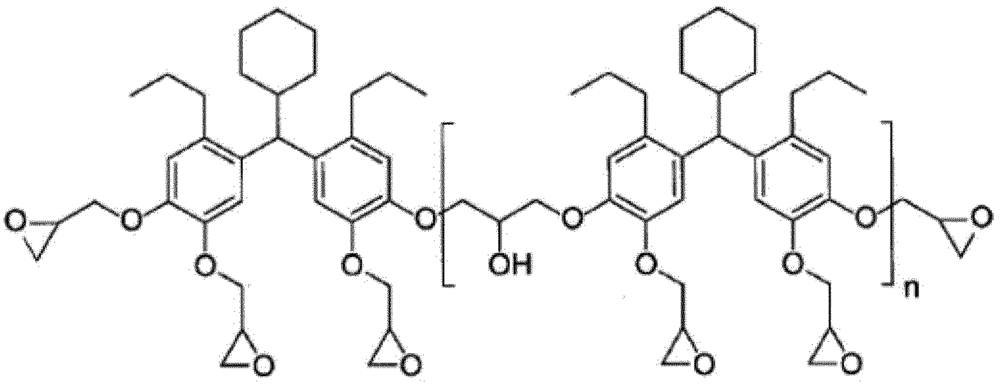

Epoxidized Bis-Propylcatechol Resin 3

・・・(19)

Briefly, bis-propylcatechol 3 obtained in the above(15.54 g, 38.99 mmol, 1.00 eq.), TBABr (1.26 g, 3.90 mmol, 0.10 eq.) and epichlorohydrin (72.16 g, 779.90 mmol, 20 eq.) were added in a four-neck round bottom flask equipped with a magnetic stirred and a condenser. The reaction was conducted at 60°C during 3 hours. Then an aqueous solution of NaOH (50w%, 4 eq) was added. The reaction was still conducted during 3 hours at 60°C. Then, deionized water was added to the mixture to dilute four times the NaOH solution and an equal volume of ethyl acetate was added. The mixture was stirred and the aqueous phase was extracted 2 more times with ethyl acetate (2x200 mL). Organic phases were combined, rinsed with brine and dried on anhydrous $MgSO_4$. Ethyl acetate and excess of epichlorohydrin were removed under vacuum. The epoxidized bis-propylcatechol resin 3 was obtained quantitatively as orange solid with a 89% yield. The EEW of epoxidized bis-propylcatechol resin 3 was titrated by $^{1}H$ NMR at 231 $g.eq^{-1}$. The measurement by the GPC method of the molecular weight (in terms of standard polystyrene) of the resulting epoxidized bis-propylcatchol resin 3 resulted in a number average molecular weight Mn of 718 $g.mol^{-1}$, a weight average molecular weight Mw of 726 $g.mol^{-1}$, and a molecular weight distribution Mw/Mn of 1.01. Accordingly, the average value of n calculated from Mn was 1.15.

The $^{1}H$ and $^{13}C$ NMR spectra of the resulting product in deuterated dimethyl sulfoxide were a follow.

$^{1}H$ NMR (400 MHz, DMSO-$d_6$) $\delta$(ppm) = 6.88-6.32 (m, 4H), 5.0 (d, 1H), 4.32 (m, 4H), 3.84 (m, 4H), 3.31 (m, 4H), 2.82 (m, 4H), 2.69 (m, 4H), 2.42-2.23 (m, 4H), 1.47 (sex, 4H), 0.98 (t, 6H), 1.71-0.71 (m, 11H).

$^{13}C$ NMR (100 MHz, DMSO-$d_6$) $\delta$(ppm) = 146.94, 145.66, 137.01, 135.00, 115.77, 115.34, 70.51, 50.37,47.01,44.94, 37.54,33.42, 31.64, 27.02, 24.61, 24.20,14.60.

• Formulation of epoxy materials

Briefly, first, the epoxy resin (epoxidized bis-propylcatechol resin 1, 2 or 3 obtained in the above) and isophoronediamine were stirred together mechanically during 2 min at 2,000 rpm with a Speed Mixer in a glass pot. The molar ratio epoxy/$NH_2$ was at 2/1. Once homogeneous mixture was obtained, the mixture was poured into a PTFE molds in order to obtain a materials with a rectangular shape for DMA analyses. The epoxy-amine thermosets were cross-linked at 100°C during 1.5 hours then 160 °C during 1 hour. Tg, Tα, shore D of the epoxy materials of Examples 1, 2, and 3 are shown in Table 1. Tg, the glass transition temperature, was measured by DSC under an argon atmosphere; Tα, the transition relaxation temperature was obtained by DMA; and hardness Shore D were measured with a durometer.

• Comparative Example

In the above, an epoxy material was obtained in the same manner as in the examples 1, 2 and 3 (formulation of epoxy material) except that DGEBA was used instead of epoxidized bis-propylcatechol. Tg, Tα, and Shore D hardness of the obtained epoxy material of Comparative Example were measured. The results are shown in Table 1.

[Table 1]

| | Tg (°C) | Tα (°C) | Shore D hardness |
|---|---|---|---|
| Example 1 | 166 | 165 | 86 |
| Example 2 | 117 | 110 | 83 |
| Example 3 | 132 | 126 | 84 |
| Comparati ve Example | 152 | 147 | 84 |

As shown in Table 1, Example 1 had higher Tg, Tα, and Shore D hardness than the comparative example. That is, it was found that the epoxy material using bis-propylcatechol of Example 1 has both thermal and mechanical properties higher than the conventional epoxy materials using DGEBA.

The Shore D hardness of Example 2 and 3, and Comparative Example were almost same. That is, it was found that the mechanical properties of the epoxy materials using bis-propylcatechol of Example 2 and 3 were comparable to the conventional epoxy materials using DGEBA.

The reason why Tg and Tα were lower in Examples 2 and 3 than in Example 1 was considered to be due to the structural difference. The bis-propylcatechol of Examples 1 to 3 is a bis-propylcatechol represented by the formula (1) in which $R^1$ is - $CHR^5$-. In Example 1, $R^5$ is 4-isopropylphenyl group (aryl group which may have a substituent), in Example 2, $R^5$ is propyl group (alkyl group), in Example 3, $R^5$ is cyclohexyl group (cycloalkyl group). It was considered that the rigidity of the aromatic ring structure allowed the bis-propylcatechol to show higher thermal properties in Example 1 where the bis-propylcatechol contained an aromatic ring in $R^1$ as a linker connecting the propylcatechol structures, as compared to the case of the bis-propylcatechols of Examples 2 and 3 that did not contain an aromatic ring.

## Claims

1. A bis-propylcatechol having a structure represented by formula (1):

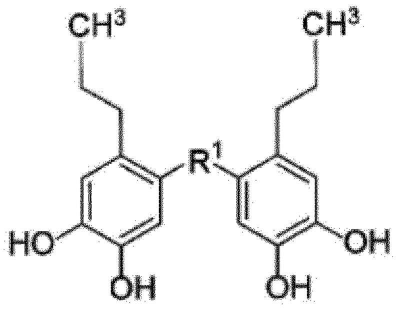

・・・(1)

wherein $R^1$ is a divalent aliphatic hydrocarbon group having two or more carbon atoms or a divalent aromatic hydrocarbon group.

2. The bis-propylcatechol according to claim 1, wherein $R^1$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

3. The bis-propylcatechol according to [1], wherein $R^1$ is a divalent hydrocarbon group represented by $-CHR^5-$ wherein $R^5$ represents a monovalent hydrocarbon group.

4. The bis-propylcatechol according to claim 1, wherein $R^1$ is a divalent aromatic hydrocarbon group represented by formula (2):

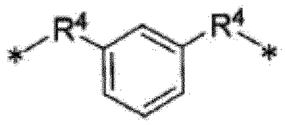

· · · (2)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure, and * represents a binding site to a benzene ring in the propylcatechol structure in the above formula (1).

**5.** The bis-propylcatechol according to claim 4, wherein each of $R^2$ and $R^3$ represents a methyl group.

**6.** The bis-propylcatechol according to claim 1 or 3, wherein $R^1$ is a divalent hydrocarbon group represented by formula (3):

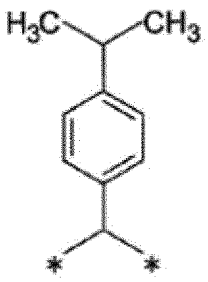

· · · (3)

wherein * represents a binding site to a benzene ring in the propylcatechol structure in the above formula (1).

**7.** The bis-propylcatechol according to claim 1, wherein $R^1$ is a divalent aliphatic hydrocarbon group represented by formula (4):

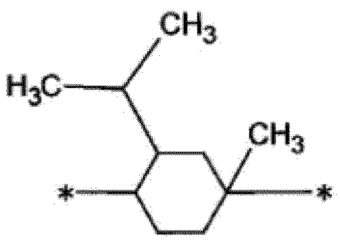

· · · (4)

wherein * represents a binding site to a benzene ring in the propylcatechol structure in the above formula (1).

**8.** The bis-propylcatechol according to claim 1 or 3, wherein $R^1$ is a divalent aliphatic hydrocarbon group represented by formula (5):

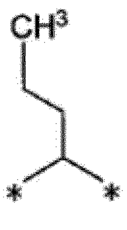

· · · (5)

wherein * represents a binding site to a benzene ring in the propylcatechol structure in the above formula (1).

**9.** The bis-propylcatechol according to claim 1 or 3, wherein $R^1$ is a divalent aliphatic hydrocarbon group represented by formula (6):

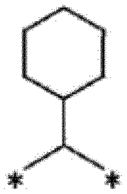

... (6)

wherein * represents a binding site to a benzene ring in the propylcatechol structure in the above formula (1).

**10.** A method for producing the bis-propylcatechol of any one of claims 1 to 9, comprising reacting a propylcatechol and at least one reactant selected from the group consisting of a ketone, an alcohol, an aldehyde and a mono-terpene.

**11.** A resin composition comprising the bis-propylcatechol according to any one of claims 1 to 9 and at least one polymer other than a bis-propylcatechol polymer.

**12.** A cured resin product, obtained by curing the resin composition according to claim 11.

**13.** An epoxidized bis-propylcatechol represented by formula (7):

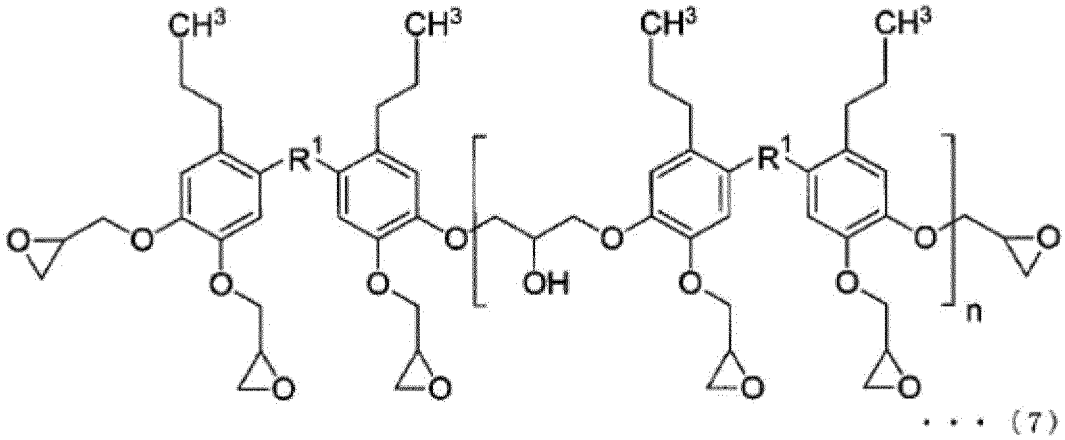

... (7)

wherein $R^1$ is a divalent aliphatic hydrocarbon group having two or more carbon atoms or a divalent aromatic hydrocarbon group, and n is an integer of 0 to 300.

**14.** The epoxidized bis-propylcatechol according to claim 13, wherein $R^1$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure.

**15.** The epoxidized bis-propylcatechol according to claim 13, wherein $R^1$ is a divalent hydrocarbon group represented by $-CHR^5-$ wherein $R^5$ represents a monovalent hydrocarbon group.

**16.** The epoxidized bis-propylcatechol according to claim 13, wherein $R^1$ is a divalent aromatic hydrocarbon group represented by formula (8):

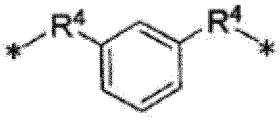

... (8)

wherein $R^4$ is a divalent hydrocarbon group represented by $-CR^2R^3-$ wherein each of $R^2$ and $R^3$ independently represents a monovalent hydrocarbon group, and $R^2$ and $R^3$ may together form a cyclic structure, and * represents a binding site to a benzene ring in the epoxidized propylcatechol structure in the above formula (7).

**17.** The epoxidized bis-propylcatechol according to claim 13 or 15, wherein $R^1$ is a divalent hydrocarbon group represented by formula (9):

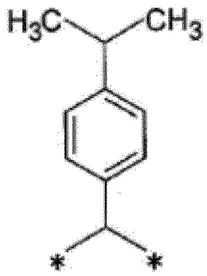

· · · (9)

wherein * represents a binding site to a benzene ring in the epoxidized propylcatechol structure in the above formula (7).

**18.** The epoxidized bis-propylcatechol according to claim 13, wherein $R^1$ is a divalent aliphatic hydrocarbon group represented by formula (10):

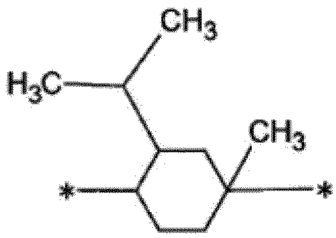

· · · (10)

wherein * represents a binding site to a benzene ring in the epoxidized propylcatechol structure in the above formula (7).

**19.** The epoxidized bis-propylcatechol according to claim 13 or 15, wherein $R^1$ is a divalent aliphatic hydrocarbon group represented by formula (11):

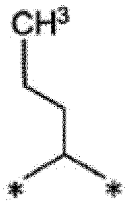

· · · (11)

wherein * represents a binding site to a benzene ring in the epoxidized propylcatechol structure in the above formula (7).

**20.** The epoxidized bis-propylcatechol according to claim 13 or 15, wherein $R^1$ is a divalent aliphatic hydrocarbon group represented by formula (12):

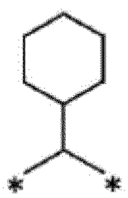

· · · (12)

wherein * represents a binding site to a benzene ring in the epoxidized propylcatechol structure in the above formula (7).

**21.** A curable resin composition comprising the epoxidized bis-propylcatechol of any one of claims 13 to 20, and a curing agent.

**22.** A method for producing the epoxidized bis-propylcatechol of any one of claims 13 to 20, comprising reacting the bis-propylcatechol of any one of claims 1 to 9 with epihalohydrin.

**23.** A cured resin product, obtained by curing the curable resin composition of claim 21.

**INTERNATIONAL SEARCH REPORT**

International application No. PCT/JP2019/011657

A. CLASSIFICATION OF SUBJECT MATTER
Int.Cl. C07C39/16(2006.01)i, C07C37/20(2006.01)i, C07C39/17(2006.01)i, C07D303/30(2006.01)i, C08G8/20(2006.01)i, C08G59/32(2006.01)i

According to International Patent Classification (IPC) or to both national classification and IPC

B. FIELDS SEARCHED

Minimum documentation searched (classification system followed by classification symbols)
Int.Cl. C07C39/16, C07C37/20, C07C39/17, C07D303/30, C08GS/20, C08G59/32

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | ZHAO, Shou; ABU-OMAR, Mahdi M., "Renewable Thermoplastics Based on Lignin-Derived Polyphenols", Macromolecules, 18 April 2017, vol. 50, no. 9, pp. 3573-3581, in particular, Scheme 1-2, page 3574, right column, line 1 to page 3575, left column, line 50, right column, lines 1-40, page 3576, right column, lines 19-29 | 1, 3, 10-13, 15, 21-23<br>2, 4-9, 14, 16-20 |
| X<br>A | JP 2014-185303 A (NAGOYA UNIVERSITY, ASAHI ORGANIC CHEMICALS INDUSTRY CO., LTD.) 02 October 2014, claim 1, paragraphs [0054]-[0065], [0111] (Family: none) | 1-6, 8-12<br>7, 13-23 |
| X<br>A | JP 4-360146 A (HITACHI CHEMICAL INDUSTRY CO., LTD.) 14 December 1992, claim 1, paragraph [0025] (Family: none) | 1, 11-12<br>2-10, 13-23 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 June 2019 (13.06.2019) | 25 June 2019 (25.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018059219 A
- JP 2018178808 A
- WO 2015061802 A1

### Non-patent literature cited in the description


- **ROCHESTER, J. R. ; BOLDEN, A. L. ; BISPHENOL S AND F.** A systematic review and comparison of the hormonal activity of Bisphenol A substitutes. *Environ Health Perspect,* 2015, vol. 123 (7), 643-50
- **ZHAO, S. ; ABU-OMAR, M. M.** Renewable Thermoplastics Based on Lignin-Derived Polyphenols. *Macromolecules,* 2017, vol. 50 (9), 3573-3581
- **ZHAO, S. ; ABU-OMAR, M. M.** Renewable epoxy networks derived from lignin-based monomers: effect of cross-linking density. *ACS Sustainable Chem. Eng.,* 2016, vol. 4 (11), 6082-6089
- **ZHUOHUA SUN et al.** Bright Side of Lignin. Depolymerization: Toward New Platform Chemicals. *Chemical Reviews,* 2018, vol. 118 (2), 614-678